# EUROPEAN PATENT APPLICATION

(11) **EP 2 050 758 A1**
(43) Date of publication of application: **22.04.2009**
(21) Application number: 07301433.4
(22) Date of filing: 04.10.2007
(51) Int. Cl.: C07K 7/08, C12N 15/10

(54) **New polypeptides and uses thereof in cancer therapy**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Warcoin, Jacques

(57) **Abstract**

The present invention concerns a polypeptide comprising (i) an amino acid sequence from less than 15 amino acids in length, which has the ability to bind to the ATPase domain of HSP70, wherein said amino acid sequence is selected in the group comprising SEQ ID NO: 9, SEQ ID NO: 18 and SEQ ID NO: 19, and derivatives thereof; a nucleic acid coding therefore; a vector comprising said nucleic acid sequence; a host cell genetically engineered with said nucleic 4 or with said vector; a pharmaceutical composition comprising said polypeptide, said nucleic acid, or said nucleic acid; and use of said polypeptide, said nucleic acid sequence, said vector, or said composition for treating cancer in a subject.

SEQUENCE LISTING

<110> Institut National de la Santé et de la Recherche Médicale
      (INSERM)

<120> New polypeptides and uses thereof in cancer therapy

<130> D25806

<160> 21

<170> PatentIn version 3.3


<210> 1
<211> 709
<212> PRT
<213> Homo sapiens

<400> 1

Leu Arg Ser Cys Cys Asp Ser Pro Leu Pro Phe Ser Arg Val Thr Pro
1               5                   10                  15
Val Val Pro Arg Leu Pro Arg Ala Asn Leu Cys Gly Cys Arg His Arg
            20                  25                  30
Arg Val Glu Phe Pro Ala Ser Gly Arg Thr Glu Leu Phe Ser Arg Ile
        35                  40                  45
Gln Cys Ser Val Ser Ser Pro Gln Ser Gln Ser Arg Ala Asp Arg Glu
    50                  55                  60
Gln Gly Thr Gly Met Ala Lys Ala Ala Ala Ile Gly Ile Asp Leu Gly
65                  70                  75                  80
Thr Thr Tyr Ser Cys Val Gly Val Phe Gln His Gly Lys Val Glu Ile
                85                  90                  95
Ile Ala Asn Asp Gln Gly Asn Arg Thr Thr Pro Ser Tyr Val Ala Phe
            100                 105                 110
Thr Asp Thr Glu Arg Leu Ile Gly Asp Ala Ala Lys Asn Gln Val Ala
        115                 120                 125
Leu Asn Pro Gln Asn Thr Val Phe Asp Ala Lys Arg Leu Ile Gly Arg
    130                 135                 140
Lys Phe Gly Asp Pro Val Val Gln Ser Asp Met Lys His Trp Pro Phe
145                 150                 155                 160
Gln Val Ile Asn Asp Gly Asp Lys Pro Lys Val Gln Val Ser Tyr Lys
                165                 170                 175
Gly Asp Thr Lys Ala Phe Tyr Pro Glu Glu Ile Ser Ser Met Val Leu
            180                 185                 190
Thr Lys Met Lys Glu Ile Ala Glu Ala Tyr Leu Gly Tyr Pro Val Thr
        195                 200                 205
Asn Ala Val Ile Thr Val Pro Ala Tyr Phe Asn Asp Ser Gln Arg Gln
    210                 215                 220
Ala Thr Lys Asp Ala Gly Val Ile Ala Gly Leu Asn Val Leu Arg Ile
225                 230                 235                 240
Ile Asn Glu Pro Thr Ala Ala Ala Ile Ala Tyr Gly Leu Asp Arg Thr
                245                 250                 255
Gly Lys Gly Glu Arg Asn Val Leu Ile Phe Asp Leu Gly Gly Gly Thr
            260                 265                 270
Phe Asp Val Ser Ile Leu Thr Ile Asp Asp Gly Ile Phe Glu Val Lys
        275                 280                 285
Ala Thr Ala Gly Asp Thr His Leu Gly Gly Glu Asp Phe Asp Asn Arg
    290                 295                 300
Leu Val Asn His Phe Val Glu Glu Phe Lys Arg Lys His Lys Lys Asp
305                 310                 315                 320

Ile Ser Gln Asn Lys Arg Ala Val Arg Arg Leu Arg Thr Ala Cys Glu
                325                 330                 335
Arg Ala Lys Arg Thr Leu Ser Ser Ser Thr Gln Ala Ser Leu Glu Ile
            340                 345                 350
Asp Ser Leu Phe Glu Gly Ile Asp Phe Tyr Thr Ser Ile Thr Arg Ala
        355                 360                 365
Arg Phe Glu Glu Leu Cys Ser Asp Leu Phe Arg Ser Thr Leu Glu Pro
    370                 375                 380
Val Glu Lys Ala Leu Arg Asp Ala Lys Leu Asp Lys Ala Gln Ile His
385                 390                 395                 400
Asp Leu Val Leu Val Gly Gly Ser Thr Arg Ile Pro Lys Val Gln Lys
                405                 410                 415
Leu Leu Gln Asp Phe Phe Asn Gly Arg Asp Leu Asn Lys Ser Ile Asn
            420                 425                 430
Pro Asp Glu Ala Val Ala Tyr Gly Ala Ala Val Gln Ala Ala Ile Leu
        435                 440                 445
Met Gly Asp Lys Ser Glu Asn Val Gln Asp Leu Leu Leu Leu Asp Val
    450                 455                 460
Ala Pro Leu Ser Leu Gly Leu Glu Thr Ala Gly Gly Val Met Thr Ala
465                 470                 475                 480
Leu Ile Lys Arg Asn Ser Thr Ile Pro Thr Lys Gln Thr Gln Ile Phe
                485                 490                 495
Thr Thr Tyr Ser Asp Asn Gln Pro Gly Val Leu Ile Gln Val Tyr Glu
            500                 505                 510
Gly Glu Arg Ala Met Thr Lys Asp Asn Asn Leu Leu Gly Arg Phe Glu
        515                 520                 525
Leu Ser Gly Ile Pro Pro Ala Pro Arg Gly Val Pro Gln Ile Glu Val
    530                 535                 540
Thr Phe Asp Ile Asp Ala Asn Gly Ile Leu Asn Val Thr Ala Thr Asp
545                 550                 555                 560
Lys Ser Thr Gly Lys Ala Asn Lys Ile Thr Ile Thr Asn Asp Lys Gly
                565                 570                 575
Arg Leu Ser Lys Glu Glu Ile Glu Ser Met Val Gln Glu Ala Glu Lys
            580                 585                 590
Tyr Lys Ala Glu Asp Glu Val Gln Arg Glu Arg Val Ser Ala Lys Asn
        595                 600                 605
Ala Leu Glu Ser Tyr Ala Phe Asn Met Lys Ser Ala Val Glu Asp Glu
    610                 615                 620
Gly Leu Lys Gly Lys Ile Ser Glu Ala Asp Lys Lys Lys Val Leu Asp
625                 630                 635                 640
Lys Cys Gln Glu Val Ile Ser Trp Leu Asp Ala Asn Thr Leu Ala Glu
                645                 650                 655
Lys Asp Glu Phe Glu His Lys Arg Lys Glu Leu Glu Gln Val Cys Asn
            660                 665                 670
Pro Ile Ile Ser Gly Leu Tyr Gln Gly Ala Gly Gly Pro Gly Pro Gly
        675                 680                 685
Gly Phe Gly Ala Gln Gly Pro Lys Gly Gly Ser Gly Ser Gly Pro Thr
    690                 695                 700
Ile Glu Glu Val Asp
705

<210> 2
<211> 8
<212> PRT
<213> Artificial sequence

<220>
<223> HSP70 binding polypeptide

<400> 2

His Thr Leu Leu Thr Pro Arg Arg
1               5

<210> 3
<211> 8
<212> PRT
<213> Artificial sequence

<220>
<223> HSP70 binding polypeptide

<400> 3

Ile Cys Leu Arg Leu Pro Gly Cys
1               5

<210> 4
<211> 8
<212> PRT
<213> Artificial sequence

<220>
<223> HSP70 binding polypeptide

<400> 4

Lys Ala Phe Trp Gly Leu Gln His
1               5

<210> 5
<211> 8
<212> PRT
<213> Artificial sequence

<220>
<223> HSP70 binding polypeptide

<400> 5

Leu Ala Leu Met Leu Pro Gly Cys
1               5

<210> 6
<211> 8
<212> PRT
<213> Artificial sequence

<220>
<223> HSP70 binding polypeptide

<400> 6

Leu Gly Phe Trp Gly Leu Pro His
1               5

<210> 7
<211> 8
<212> PRT
<213> Artificial sequence

<220>
<223> HSP70 binding polypeptide

<400> 7

Leu Val Pro Cys Leu Pro Gly Cys
1               5


<210> 8
<211> 8
<212> PRT
<213> Artificial sequence

<220>
<223> HSP70 binding polypeptide

<400> 8

Arg Ala Leu Trp Gly Leu Gln His
1               5


<210> 9
<211> 8
<212> PRT
<213> Artificial sequence

<220>
<223> HSP70 binding polypeptide

<400> 9

Ser Pro Trp Pro Arg Pro Thr Tyr
1               5


<210> 10
<211> 13
<212> PRT
<213> Artificial sequence

<220>
<223> HSP70 binding polypeptide

<400> 10

Ala Lys Trp Val Gly Asp Leu Thr Leu Cys Arg Trp Arg
1               5                   10

<210> 11
<211> 13
<212> PRT
<213> Artificial sequence

<220>

<223> HSP70 binding polypeptide

<400> 11

Cys Ile Pro Met Ala Trp Ala Val Ser Trp Pro His Pro
1               5                   10


<210> 12
<211> 13
<212> PRT
<213> Artificial sequence

<220>
<223> HSP70 binding polypeptide

<400> 12

Cys Ile Trp Val Ser Asp Gly Lys Lys Leu Trp Arg His
1               5                   10


<210> 13
<211> 13
<212> PRT
<213> Artificial sequence

<220>
<223> HSP70 binding polypeptide

<400> 13

Cys Tyr Thr Gln Tyr Arg Lys Cys Gln Glu Leu Thr Ala
1               5                   10


<210> 14
<211> 13
<212> PRT
<213> Artificial sequence

<220>
<223> HSP70 binding polypeptide

<400> 14

Glu Val Trp Arg Leu Ala Glu Phe Leu Ala Met Pro Pro
1               5                   10


<210> 15
<211> 13
<212> PRT
<213> Artificial sequence

<220>
<223> HSP70 binding polypeptide

<400> 15

Ile Ala Ala His Asp Thr Pro Gly Pro Val Trp Leu Ser

1               5                   10


<210> 16
<211> 13
<212> PRT
<213> Artificial sequence

<220>
<223> HSP70 binding polypeptide

<400> 16

Pro Asn Glu Val Asn Arg Leu Ala His Leu Arg Leu His
1               5                   10


<210> 17
<211> 13
<212> PRT
<213> Artificial sequence

<220>
<223> HSP70 binding polypeptide

<400> 17

Ser Pro Leu Gly Tyr Gly Phe Ala Val Arg Asn Ser Gly
1               5                   10


<210> 18
<211> 13
<212> PRT
<213> Artificial sequence

<220>
<223> HSP70 binding polypeptide

<400> 18

Val Gly Gln Ile Val Pro Tyr Gly Ser Cys Thr His Ala
1               5                   10


<210> 19
<211> 13
<212> PRT
<213> Artificial sequence

<220>
<223> HSP70 binding polypeptide

<400> 19

Tyr Cys Ala Tyr Tyr Ser Pro Arg His Lys Thr Thr Phe
1               5                   10


<210> 20
<211> 380

<212> PRT
<213> homo sapiens

<400> 20

Lys Ala Ala Ala Ile Gly Ile Asp Leu Gly Thr Thr Tyr Ser Cys Val
1               5                   10                  15
Gly Val Phe Gln His Gly Lys Val Glu Ile Ile Ala Asn Asp Gln Gly
            20                  25                  30
Asn Arg Thr Thr Pro Ser Tyr Val Ala Phe Thr Asp Thr Glu Arg Leu
        35                  40                  45
Ile Gly Asp Ala Ala Lys Asn Gln Val Ala Leu Asn Pro Gln Asn Thr
    50                  55                  60
Val Phe Asp Ala Lys Arg Leu Ile Gly Arg Lys Phe Gly Asp Pro Val
65                  70                  75                  80
Val Gln Ser Asp Met Lys His Trp Pro Phe Gln Val Ile Asn Asp Gly
                85                  90                  95
Asp Lys Pro Lys Val Gln Val Ser Tyr Lys Gly Glu Thr Lys Ala Phe
            100                 105                 110
Tyr Pro Glu Glu Ile Ser Ser Met Val Leu Thr Lys Met Lys Glu Ile
        115                 120                 125
Ala Glu Ala Tyr Leu Gly Tyr Pro Val Thr Asn Ala Val Ile Thr Val
    130                 135                 140
Pro Ala Tyr Phe Asn Asp Ser Gln Arg Gln Ala Thr Lys Asp Ala Gly
145                 150                 155                 160
Val Ile Ala Gly Leu Asn Val Leu Arg Ile Ile Asn Glu Pro Thr Ala
                165                 170                 175
Ala Ala Ile Ala Tyr Gly Leu Asp Arg Thr Gly Lys Gly Glu Arg Asn
            180                 185                 190
Val Leu Ile Phe Asp Leu Gly Gly Gly Thr Phe Asp Val Ser Ile Leu
        195                 200                 205
Thr Ile Asp Asp Gly Ile Phe Glu Val Lys Ala Thr Ala Gly Asp Thr
    210                 215                 220
His Leu Gly Gly Glu Asp Phe Asp Asn Arg Leu Val Asn His Phe Val
225                 230                 235                 240
Glu Glu Phe Lys Arg Lys His Lys Lys Asp Ile Ser Gln Asn Lys Arg
                245                 250                 255
Ala Val Arg Arg Leu Arg Thr Ala Cys Glu Arg Ala Lys Arg Thr Leu
            260                 265                 270
Ser Ser Ser Thr Gln Ala Ser Leu Glu Ile Asp Ser Leu Phe Glu Gly
        275                 280                 285
Ile Asp Phe Tyr Thr Ser Ile Thr Arg Ala Arg Phe Glu Glu Leu Cys
    290                 295                 300
Ser Asp Leu Phe Arg Ser Thr Leu Glu Pro Val Glu Lys Ala Leu Arg
305                 310                 315                 320
Asp Ala Lys Leu Asp Lys Ala Gln Ile His Asp Leu Val Leu Val Gly
                325                 330                 335
Gly Ser Thr Arg Ile Pro Lys Val Gln Lys Leu Leu Gln Asp Phe Phe
            340                 345                 350
Asn Gly Arg Asp Leu Asn Lys Ser Ile Asn Pro Asp Glu Ala Val Ala
        355                 360                 365
Tyr Gly Ala Ala Val Gln Ala Ala Ile Leu Met Gly
    370                 375                 380


<210> 21
<211> 13
<212> PRT
<213> Artificial sequence

<220>

<223> Control peptide

<400> 21

His His Met Gln Ala His Ala Met Leu Gly Arg Arg Arg
1               5                   10

## Description

### Field of the invention

The invention relates to the field of cancer, and in particular to new polypeptides and uses thereof in cancer therapy.

### Background of the Invention

Apoptosis -i.e., programmed cell death- is responsible for the removal of unwanted or supernumerary cells during development, as well as in adult homeostasis (JACOBSON et al., Cell, vol.88, p:347-354, 1997). Apoptosis is also the predominant form of cell death triggered by cytotoxic drugs in tumor cells (SOLARY et al., Leukemia, vol.14, p:1833-1849, 2000). The problem for treating cancer is that apoptotic program is frequently disabled in tumors. As a result, these cells are particularly resistant to the induction of cell death by chemotherapeutic agents.

Drugs that target HSPs have recently emerged as potential anticancer agents since several of these proteins are key mediators of the cellular response to damage induced by environmental stressors (PARCELLIER et al., Biochem. Biophys. Res. Commun, vol.304, p:505-12, 2003). The only drugs so far available are inhibitors of HSP90, most of them geldanamycin derivatives like the 17-allylamino,17-demethoxygeldanamycin (17AAG), currently in phase I and II clinical trials. Unfortunately, no drugs are available to neutralize stress-inducible heat shock protein 70 (HSP70), which is a prominent cytoprotective factor.

Under normal conditions, HSP70 functions as an ATP-dependent chaperone by assisting the folding of newly synthesized proteins and polypeptides, the assembly of multiprotein complexes and the transport of proteins across cellular membranes (SHI & THOMAS, Mol. Cell. Biol., vol.12, p: 2186-92, 1992; MURAKAMI et al., J. Cell. Biol., vol.107, p:2051-7, 1988; BECKMANN et al., Science, vol.248, p:850-4, 1990). HSP70 upregulation, either by cellular stress or by transfection, inhibits apoptosis induced by a wide range of insults and may contribute to oncogenic transformation (EO et al., Biochem. Biophys. Res. Commun, vol.218, p:582-7, 1996; VOLLOCH & SHERMAN, Oncogene, vol.18, p:3648-51, 1999). Thus, HSP70 overexpression increases the tumorigenicity of cancer cells in rodent models (JAATTELA, Int. J. Cancer, vol.60, p:689-93, 1995), and correlates with poor prognosis in breast cancer (VARGAS-ROIG et al., Int. J. Cancer, vol.79, p:468-75, 1998). Conversely, HSP70 downregulation is sufficient to kill tumor cells or to facilitate the induction of apoptosis *in vitro* (NYLANDSTED et al., Proc. Natl. Acad. Sci. U S A, vol.97, p:7871-6, 2000) and reduced tumorigenicity *in vivo* (GURBUXANI et al., Oncogene, vol.20, p:7478-85, 2001). The anti-apoptotic function of HSP70 involves interactions with several components of the apoptotic machinery. HSP70 has been demonstrated to bind to Apaf-1, thereby preventing the recruitment of procaspase-9 to the apoptosome (BEERE et al., Nat. Cell Biol., vol.2, p:469-75, 2000). It has been also shown that HSP70 can also inhibit apoptosis by directly interacting with and neutralizing apoptosis inducing factor (AIF; RAVAGNAN et al., Nat. Cell Biol., vol.3, p:839-43, 2001).

### Summary of the invention

In a first aspect, the present invention relates to a polypeptide comprising (i) an amino acid sequence from less than 15 amino acids in length, which has the ability to bind to HSP70, wherein said amino acid sequence is selected in the group comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19, and derivatives thereof.

In a second aspect the present invention relates to a nucleic acid encoding for a polypeptide as described above, wherein said nucleic acid may be delivered *in vivo* alone or in association with a vector.

In a third aspect, the present invention relates to a host cell genetically engineered with the polynucleotide or with the vector described previously

In a forth aspect, the present invention relates to a composition comprising a polypeptide as described above, a nucleic acid encoding thereof, or a nucleic acid vector comprising said nucleic acid, eventually associated with a pharmaceutically acceptable vehicle.

In a fifth aspect, the present invention relates to the use of a polypeptide as describe above, a nucleic acid sequence coding therefore, a vector comprising such a nucleic acid sequence, or a composition as described previously for treating cancer in a subject.

In a sixth aspect, the present invention relates to the products containing:
(i) a polypeptide as describe above, a nucleic acid sequence coding therefore, or a vector comprising such a nucleic acid sequence, and
(ii) a cytotoxic agent,
as a combined preparation for simultaneous, separate, or sequential use for treating cancer in a subj ect.

In a seventh aspect, the present invention relates to a method for treating cancer comprising administrating to a subject a therapeutically effective amount of a polypeptide as describe above, a nucleic acid sequence coding therefore, a vector comprising such a nucleic acid sequence, or a composition as described previously.

In a eighth aspect, the present invention relates to a method for treating cancer comprising the step of simultaneously, separately, or sequentially administrating to a subject in need thereof a therapeutically effective amount of:
(i) a polypeptide as describe above, a nucleic acid sequence coding therefore, or a vector comprising such a nucleic acid sequence, and
(ii) a cytotoxic agent.

### Brief description of the drawings

Figures 1A and 1B show the interaction of the peptide aptamers with endogenous HSP70 in Hela cells.
Figures 2A and 2B show the cell sensitization to apoptosis death with A8 and A18 aptamers.
Figures 3A and 3B show the specific binding of A8 and A18 aptamers to the ATPase domain of HSP70.
Figures 4A and 4B show the tumour size decrease associated with A8 and A18 aptamers expression.

### Description of the Preferred embodiments

In a first aspect, the present invention relates to a polypeptide comprising (i) an amino acid sequence from less than 15 amino acids in length, which has the ability to bind to HSP70, wherein said amino acid sequence is selected in the group comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19, and derivatives thereof, preferably in the group comprising SEQ ID NO: 5, SEQ ID NO: 6, SEQ ID NO: 7, SEQ ID NO: 9, SEQ ID NO: 10, SEQ ID NO: 11, SEQ ID NO: 14, SEQ ID NO: 15, SEQ ID NO: 17, SEQ ID NO: 18 and SEQ ID NO: 19, and derivatives thereof.

According to the present invention, the length of the polypeptide of the invention is less than 50 amino acids, preferably less than 25 amino acids.

Preferably, HSP 70 refers to SEQ ID NO:1.

In a preferred embodiment, the amino acid sequence from less than 15 amino acids which has the ability to bind HSP70 in the group comprising SEQ ID NO: 6, SEQ ID NO: 9, SEQ ID NO: 17, SEQ ID NO: 18 and SEQ ID NO: 19, and derivatives thereof., preferably in the group comprising SEQ ID NO: 9, SEQ ID NO: 18 and SEQ ID NO: 19, and derivatives thereof.

In still another preferred embodiment, the amino acid sequence from less than 15 amino acids has the ability to bind to the ATPase domain of HSP70, and is selected in the group comprising SEQ ID NO: 9, and SEQ ID NO: 19, and derivatives thereof.

Preferably, the ATPase domain of HSP70 refers to SEQ ID NO:20.

Advantageously, the amino acid sequence having the ability to bind to HSP70 (SEQ ID NO:1) is more than 6 amino acids in length, preferably more than or equal to 8 amino acid in length.

More advantageously, the amino acid sequence having the ability to bind to HSP70 is less than or equal to 13 amino acid in length.

As used herein an amino acid sequence having the ability to bind to HSP70, and preferably to the ATPase domain of HSP70, can simply be identified by one of skill in the art in view of the following examples. As an example, the skilled person can screen a peptide library for identifying by double-hybrid the peptides which bind to HSP70, and preferably to the ATPase domain of HSP70. Such an amino acid sequence which binds to HSP70, and preferably to the ATPase domain of HSP70, has typically an affinity of less or equal to 10⁻⁶ M, preferably less or equal to 10⁻⁹ M for HSP70 or for the ATPase domain of HSP70 respectively.

As used herein, the term "derivatives"' refer to an amino acid sequence having a percentage of identity of at least 80% with an amino acid sequence from less than 15 amino acids in length having the ability to bind to the ATPase domain of HSP70, wherein said amino acid sequence is selected in the group comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19, preferably of at least 90%.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously align according to the best alignment; this comparison is realized on segments of comparison in order to identify and compared the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developed by NEDDLEMAN and WUNSCH (J. Mol. Biol., vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p:1792, 2004 ). To get the best local alignment, one can preferably used BLAST software, with the BLOSUM 62 matrix, or the PAM 30 matrix. The identity percentage between two sequences of amino acids is determined by comparing these two sequences optimally aligned, the amino acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

It will also be understood that natural amino acids may be replaced by chemically modified amino acids. Typically, such chemically modified amino acids enable to increase the polypeptide half life.

According to another preferred embodiment, the polypeptide of the invention also comprises (ii) an amino acid sequence coding for a cell penetrating peptide.

The term "cell penetrating peptide" (CPP) is defined as a carrier peptide that is capable of crossing biological membrane or a physiological barrier. Cell penetrating peptides are also called cell-permeable peptides, protein-transduction domains (PTD) or membrane- translocation sequences (MTS). CPPs have the ability to translocate in vitro and/or in vivo the mammalian cell membranes and enter into cells, and directs the apoptosis domain of interest to the cytoplasm.

Several proteins and their peptide derivatives have been found to possess cell internalization properties including but not limited to the Human Immunodeficency Virus type 1 (HIV-I) protein Tat (RUBEN et al., J. Virol., vol.63, p:1-8, 1989), the herpes virus tegument protein VP22 (ELLIOTT and O'HARE, Cell, vol.88, p:223-233, 1997)), the homeotic protein of Drosophila melanogaster Antennapedia (the CPP is called Penetratin) (DEROSSI et al., J. Biol. Chem., vol.271, p:18188-18193, 1996), the protegrin 1 (PG-I) anti-microbial peptide SynB (KOKRYAKOV et al, FEBS Lett., vol.327, p:231-236, 1993) and the basic fibroblast growth factor (JANS, Faseb J., vol.8, p:841-847, 1994). A number of other proteins and their peptide derivatives have been found to possess similar cell internalization properties such as the peptides disclosed in patent applications WO 01/64738 and EP1512696. As general references on CPPs it can be cited: CELL PENETRATING PEPTIDES: PROCESSES AND APPLICATIONS, edited by UIO LANGEL (2002); or Advanced Drug Delivery Reviews (vol.57, p:489-660, 2005); or DIETZ and BAHR (Moll. Cell. Neurosci., vol.27, p:85-131, 2004).

In a second aspect the present invention relates to a nucleic acid encoding for a polypeptide as described above.

Said nucleic acid corresponds to RNA or DNA, preferably to DNA.

According to a preferred embodiment, the nucleic acid encoding the polypeptide of the invention is operatively linked to a gene expression sequence, which directs the expression of nucleic acid within a prokarotic or an eukaryotic cell, preferably within an eukaryotic cell. The "gene expression sequence" is any regulatory nucleotide sequence, such as a promoter sequence or promoter-enhancer combination, which facilitates the efficient transcription and translation of the peptidic antagonist nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, beta.-actin promoter, muscle creatine kinase promoter, human elongation factor promoter and other constitutive promoters. Exemplary viral promoters which function constitutively in eukaryotic cells include, for example, promoters from the simian virus (e.g., SV40), papilloma virus, adenovirus, human immunodeficiency virus (HIV), cytomegalovirus (CMV), Rous sarcoma virus (RSV), hepatitis B virus (HBV), the long terminal repeats (LTR) of Moloney leukemia virus and other retroviruses, and the thymidine kinase promoter of herpes simplex virus. Others constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Others inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired.

As used herein, the nucleic acid sequence encoding the polypeptide of the invention and the gene expression sequence are said to be "operably linked" when they are covalently linked in such a way as to place the expression or transcription and/or translation of the polypeptide of the invention coding sequence under the influence or control of the gene expression sequence. Two DNA sequences are said to be operably linked if induction of a promoter in the 5' gene expression sequence results in the transcription of the polypeptide of the invention and if the nature of the linkage between the two DNA sequences does not (1) result in the introduction of a frame-shift mutation, (2) interfere with the ability of the promoter region to direct the transcription of the polypeptide of the invention, or (3) interfere with the ability of the corresponding RNA transcript to be translated into a protein. Thus, a gene expression sequence would be operably linked to a nucleic acid sequence coding for the polypeptide of the invention if the gene expression sequence were capable of effecting transcription of that nucleic acid sequence such that the resulting transcript is translated into the desired polypeptide.

The nucleic acid coding for the polypeptide of the invention may be delivered *in vivo* alone or in association with a vector. In its broadest sense, a "vector" is any vehicle capable of facilitating the transfer of the nucleic acid coding for the polypeptide of the invention to the cells. Preferably, the vector transports the nucleic acid to cells with reduced degradation relative to the extent of degradation that would result in the absence of the vector. In general, the vectors useful in the invention include, but are not limited to, plasmids, phagmids, viruses, other vehicles derived from viral or bacterial sources that have been manipulated by the insertion or incorporation of the peptidic antagonist nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in KRIEGLER (A Laboratory Manual," W.H. Freeman C.O., New York, 1990) and in MURRY ("Methods in Molecular Biology," vol.7, Humana Press, Inc., Cliffton, N.J., 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g., SANBROOK et al., "Molecular Cloning: A Laboratory Manual," Second Edition, Cold Spring Harbor Laboratory Press, 1989. In the last few years, plasmid vectors have been used as DNA vaccines for delivering antigen-encoding genes to cells *in vivo*. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, intratumoral or other routes. The plasmids may be given in an aqueous solution, dried onto gold particles or in association with another DNA delivery system including but not limited to liposomes, dendrimers, cochleate and microencapsulation.

The nucleic acid vector can include selectable markers that are active both in bacteria and in mammalian cells.

According to a first specific embodiment, the nucleic acid vector of the present invention corresponds to "naked DNA" like plasmids, cosmids or phagemids. Such naked DNA can be associated with non-lipid cationic polymers (EP 0770440, WU and WU, J. Biol. Chem., vol.263, p: 14621-4, 1988) or liposomes (BRIGHMAN et al., Am. J. Med. Sci., vol.298, p: 278-81, 1989) to form complexes enhancing cellular uptake.

According to a second specific embodiment, the nucleic acid vector is a viral vector adapted for *in vivo* gene therapy protocols. Examples of appropriate viral vectors includes retroviral vectors as described in EP 0871459, EP 0386882 and EP 1222300 and adenovirus vectors as described in US 2004/ 265273 and US 6,638,502. In this case, the internalization of virus occurs through the specific interaction of the viral envelope with a cell surface receptor, followed by receptor-mediated endocytosis of the virus/receptor complex.

In a third aspect, the present invention relates to a host cell genetically engineered with the polynucleotide or with the vector described previously

As used herein, the term "host cell genetically engineered" relates to host cells which have been transduced, transformed or transfected with the polynucleotide or with the vector described previously.

As representative examples of appropriate host cells, one can cites bacterial cells, such as E. coli, Streptomyces, Salmonella typhimurium, fungal cells such as yeast, insect cells such as Sf9, animal cells such as CHO or COS, plant cells, etc. The selection of an appropriate host is deemed to be within the scope of those skilled in the art from the teachings herein.

The introduction of the polynucleotide or of the vector described previously into the host cell can be effected by method well known from one of skill in the art such as calcium phosphate transfection, DEAE-Dextran mediated transfection, or electroporation.

In a forth embodiment, the present invention relates to a composition comprising a polypeptide as described above, a nucleic acid encoding thereof, or a nucleic acid vector comprising said nucleic acid, eventually associated with a pharmaceutically acceptable vehicle.

According to a specific embodiment, the composition of the invention further comprises at least one cytotoxic agent.

As used herein, the expression "cytotoxic agent" refers to a chemical compound that is directly toxic to cells by inducing apoptosis and which is useful or used in cancer therapy. As an example of such cytotoxic agent, one can cite 5-FU, cisplatin, methotrexate, oxaliplatin, etoposide, doxorubicin, daunorubycin, or melphalan.

The inventors have established that the administration of the polypeptides of the invention increases tumor cells susceptibility *in vivo* and *in vitro* to cytotoxic agent.

According to another specific embodiment, the composition of the invention do not comprises any cytotoxic agent.

In fact, the inventors have also established that the administration of the polypeptides of the invention without any cytotoxic agent administration is able to induce an increase of the number of tumor infiltrating CD8+ T cells and of macrophages, said increase resulting in a decrease of tumor progression *in vivo.*

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

The polypeptides of the invention, nucleic acids coding therefore or nucleic acid vectors may be solubilized in a buffer or water or incorporated in emulsions and microemulsions. Suitable buffers include, but are not limited to, phosphate buffered saline Ca⁺⁺/Mg⁺⁺ free (PBS), phosphate buffered saline (PBS), normal saline (150 mM NaCl in water), Tris buffer and surfactants.

There are numerous causes of peptide instability or degradation, including hydrolysis and denaturation. Hydrophobic interaction may cause clumping of molecules together (i.e. aggregation). This result may entail diminution of the induction of a Treg response. Stabilizers may be added to lessen or prevent such problems.

Stabilizers include cyclodextrine and derivatives thereof (see U.S. Pat. No.5,730,969). Suitable preservatives such as sucrose, mannitol, sorbitol, trehalose, dextran and glycerin can also be added to stabilize the final formulation. A stabilizer selected from ionic and non-ionic surfactants, D-glucose, D-galactose, D-xylose, D-galacturonic acid, trehalose, dextrans, hydroxyethyl starches, and mixtures thereof may be added to the formulation. Addition of alkali metal salt or magnesium chloride may stabilize a peptide. The peptide may also be stabilized by contacting it with a saccharide selected from the group consisting of dextran, chondroitin sulphuric acid, starch, glycogen, dextrin, and alginic acid salt. Other sugars that can be added include monosaccharides, disaccharides, sugar alcohols, and mixtures thereof (E.g., glucose, mannose, galactose, fructose, sucrose, maltose, lactose, mannitol, xylitol). Polyols may stabilize a peptide, and are water-miscible or water-soluble. Suitable polyols may be polyhydroxy alcohols, monosaccharides and disaccharides including mannitol, glycrol, ethylene glycol, propylene glycol, trimethyl glycol, vinyl pyrrolidone, glucose, fructose, arabinose, mannose, maltose, sucrose, and polymers thereof. Various excipients may also stabilize peptides, including serum albumin, amino acids, heparin, fatty acids and phospholipids, surfactants, metals, polyols, reducing agents, metal chelating agents, polyvinyl pyrrolidone, hydrolysed gelatin, and ammonium sulfate.

In a fifth aspect, the present invention relates to the use of a polypeptide as describe above, a nucleic acid sequence coding therefore, a vector comprising such a nucleic acid sequence, or a composition as described previously for treating cancer in a subject.

In fact, the apoptosis activity of the polypeptide of the invention enables to induce the death of the cells wherein they are internalized.

As used herein, the term "subject" denotes a Mammal, such as a rodent, a feline, a canine and a primate, and most preferably a human.

In a sixth aspect, the present invention relates to the products containing:
(i) a polypeptide as describe above, a nucleic acid sequence coding therefore, or a vector comprising such a nucleic acid sequence, and
(ii) a cytotoxic agent,
as a combined preparation for simultaneous, separate, or sequential use for treating cancer in a subj ect.

In a seventh aspect, the present invention relates to a method for treating cancer comprising administrating to a subject a therapeutically effective amount of a polypeptide as describe above, a nucleic acid sequence coding therefore, a vector comprising such a nucleic acid sequence, or a composition as described previously.

According to the present invention, an "effective amount" of a composition is one which is sufficient to achieve a desired biological effect, in this case inducing apoptosis in tumor cells. It is understood that the effective dosage will be dependent upon the age, sex, health, and weight of the recipient, kind of concurrent treatment, if any, frequency of treatment, and the nature of the effect desired. The ranges of effective doses provided below are not intended to limit the invention and represent preferred dose ranges. However, the preferred dosage can be tailored to the individual subject, as is understood and determinable by one of skill in the art, without undue experimentation.

In a eighth aspect, the present invention relates to a method for treating cancer comprising the step of simultaneously, separately, or sequentially administrating to a subject in need thereof a therapeutically effective amount of:
(i) a polypeptide as describe above, a nucleic acid sequence coding therefore, or a vector comprising such a nucleic acid sequence, and
(ii) a cytotoxic agent.

In a ninth aspect, the present invention relates to a method for screening candidate compounds capable of modulating the intracellular interaction between HSP70 and a polypeptide as described previously, wherein said method comprises the steps of:
(i) transforming a mammalian cell with an interactive fusion protein construct comprising a constitutive promoter operably linked to an HSP 70 coding sequence fused in frame with a DNA binding domain, a second interactive fusion protein construct comprising an inducible promoter operably linked to a sequence coding for any of the polypeptide described previously fused in frame with a transcriptional activation domain and a reporter sequence construct comprising a DNA binding element bindable to said DNA binding domain protein operably linked to a minimal promoter and a reporter coding sequence;
(ii) contacting or not a candidate compound with said mammalian cell allowing its contact with the HSP 70 coding sequence,
(iii) inducing the expression of the second interactive fusion protein corresponding to a polypeptide as described previously fused in frame with a transcriptional activation domain,
(iv) comparing the expression of the reporter coding sequence in the presence or absence of the contacting step with the candidate compound, and
(v) selecting the candidate compound which induces an inhibition of the expression of the reporter coding sequence.

The selected candidate compounds can then be tested for their ability to bind to HSP 70, and further for their ability to potential chemosensitizing and /or antitumor effects. Such effects can be assessed by example with the protocols disclosed in the example.

The mammalian cell is preferably a yeast cell.

The HSP 70 coding sequence, such as SEQ ID NO:1, corresponds preferably to the HSP 70 ATPase domain, such as SEQ ID NO: 20.

DNA binding domains are well known from one of skill in the art. As an example, one can cite the DNA binding domain of LexA.

One of skill in the art can simply realize the screening method of the invention in view of his general knowledge. As an example, the skilled person can follow the protocols disclosed in international patent application PCT WO 2005/095645, which is herein incorporated by reference from page 3, line 30 to page 17, line 33.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### EXAMPLES

### 1) Identification of HSP70-binding aptamers

The optimized yeast two-hybrid procedure disclosed in BICKLE et al. (Nat. Protoc., viol.1, p:1066-91, 2006) has been used to select peptide aptamers for their ability to bind to HSP70. For the two-hybrid screening, the human HSP70 has been used in combination with two peptide aptamer libraries consisting of a *E*.*coli* thioredoxin scaffold displaying variable peptide loops of 13 or 8 amino acids and 18 different peptide aptamers have been obtained (8 and 10 aptamers with 8 and 13 aminoacid variable regions, respectively) which are disclosed in the following Table I.

**Table I: aptamers interacting with human HSP 70**

| Peptide aptamers | Sequence (AA) | *lacZ* phenotype |
|---|---|---|
| A1 | HTLLTPRR (SEQ ID NO : 2) | + |
| A2 | ICLRLPGC (SEQ ID NO : 3) | + |
| A3 | KAFWGLQH (SEQ ID NO : 4) | ++ |
| A4 | LALMLPGC (SEQ ID NO : 5) | +/- |
| A5 | LGFWGLPH (SEQ ID NO : 6) | ++ |
| A6 | LVPCLPGC (SEQ ID NO : 7) | + |
| A7 | RALWGLQH (SEQ ID NO : 8) | ++ |
| A8 | SPWPRPTY (SEQ ID NO : 9) | + |
| A9 | AKWVGDLTLCRWR (SEQ ID NO : 10) | +/- |
| A10 | CIPMAWAVSWPHP (SEQ ID NO : 11) | + |
| A11 | CIWVSDGKKLWRH (SEQ ID NO : 12) | + |
| A12 | CYTQYRKCQELTA (SEQ ID NO : 13) | + |
| A13 | EVWRLAEFLAMPP (SEQ ID NO : 14) | + |
| A14 | IAAHDTPGPVWLS (SEQ ID NO : 15) | + |
| A15 | PNEVNRLAHLRLH (SEQ ID NO : 16) | + |
| A16 | SPLGYGFAVRNSG (SEQ ID NO : 17) | ++ |
| A17 | VGQIVPYGSCTHA (SEQ ID NO : 18) | + |
| A18 | YCAYYSPRHKTTF (SEQ ID NO : 19) | + |

### 2) Interaction of the aptamers with endogenous HSP70 in cancer cells

To determine the capacity of the selected aptamers to bind endogenous HSP70 in the cells, the aptamer coding sequences have been cloned into a HA-tagged pcDNA3 vector (INVITROGEN) according to the manufacturer's instruction.

HeLa cells were transiently transfected with HA-tagged pcDNA3 vector comprising each aptamer coding sequence -i.e., A0 (control; SEQ ID NO: 21: HHMQAHAMLGRRR) and A1 to A18- or with the empty vector as a control using the SUPERFECT REAGENT (QUIAGEN). Transfected HeLa cell extracts were prepared by lysing the cells with an immunoprecipitation buffer (50 mM Hepes (pH 7,6), 150 mM NaCl, 5 mM EDTA and 0,1 % NP40). The protein concentration was measured in the supernatant by using DC PROTEIN ASSAY (BIORAD). The supernatants were then incubated with a mouse polyclonal anti-HA tag antibody (INVITROGEN, 1:100) with a constant agitation at 4°C according to the manufacturer's instruction. The immunocomplexes were precipitated with mixed protein G and A-sepharose. The pellet was washed four times and prepared for immunobloting.

The proteins were then separated in a 14 % SDS-polyacrylamide gel and electroblotted to PVDF membranes (AMERSHAM HYBOND P). After blocking non-specific binding sites with 8 % non-fat milk in T-PBS (PBS, TWEEN 20 0.1 %), blots were incubated with a mouse anti-Hsp70 monoclonal antibody (ABCAM) according to the manufacturer's instruction. washed in T-PBS, incubated 30 min at room temperature with horseradish peroxidase-conjugated goat anti-mouse antibody (Jackson ImmunoResearch Laboratories) and revealed following the ECL Western blotting analysis procedure (AMERSHAM). All the immunoprecipitation and western blot analyses were repeated three times.

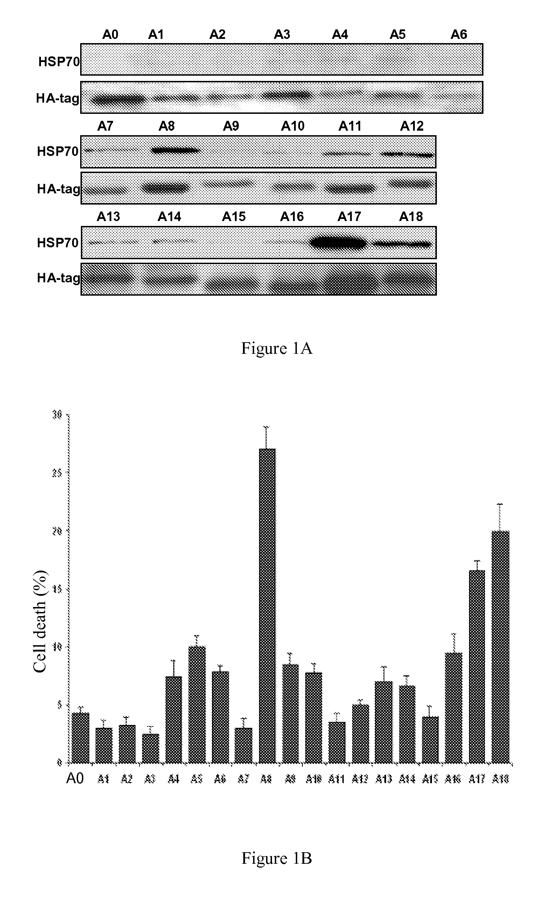

The figure 1A corresponds to the immunoblots showing the signal obtained for HSP70 and for the HA-tag with each aptamer (A0 to A18).

The results show that HSP70 was co-immunoprecipitated to various extents with most peptide aptamers. Nevertheless, five peptide aptamers showed a strong apparent binding affinity for HSP70 (A8, A11, A12, A17 and A18; see Figure1).

### 3) in vitro chemo-sensitizing properties of the aptamers

2,5.10⁵ adherent Hela cells were plated onto 6-well culture plates in complete medium. The cells were transiently transfected with HA-tagged pcDNA3 vector comprising each aptamer coding sequence as described previously and then treated with cisplatin (CDDP, 25 µM) for 24 h. The cell death was measured by the crystal violet colorimetric assay and/or after Hoechst 33342 (SIGMA) staining. The results obtained represent the mean of three independent determinations.

The figure 1B shows the percentage of cell death assessed by the use of a vital dye (X±SD, n=4).

The results established that none of the aptamers by their own showed any toxicity for the cells (not shown). However, in combination with cisplatin, 3 aptamers (A8, A17 and A18) strongly sensitized to cell death (see Figure 1B). Interestingly, A8, A17 and A18 belong to the group of peptide aptamers that showed the highest apparent binding affinity for HSP70 (see Figure 1A).

To confirm the effect of A8, A17 and A18 aptamers on cell death, Hela cells or B16F10 melanoma cells were either mock transfected or transiently transfected with A0, A8, A17 or A18 expression vectors as described previously. Then, the cells were treated with cisplatin as above dislosed, and the percentage of cells with condensate nucleus was determined by staining with Hoechst 33342.

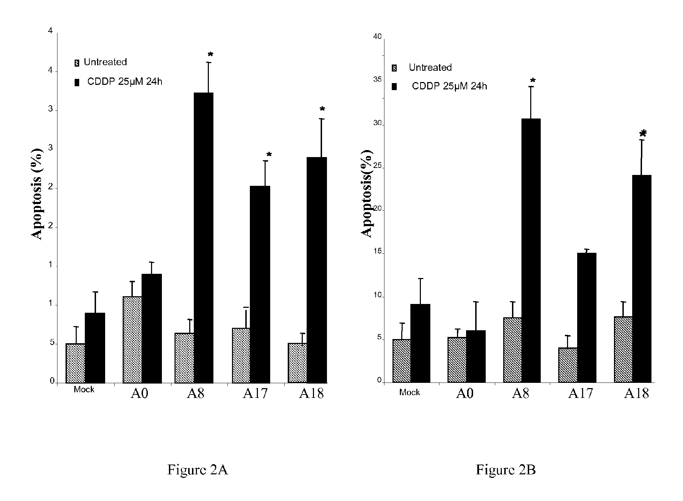

The figure 2A and 2B show the percentage of apoptosis as measured by counting respectively Hela cells or B16F10 melanoma cells having condensed and fragmented nuclear chromatin after cell staining with Hoechst 33342 dye.

Again, the results confirm that the A8, A17 and A18 aptamers alone did not have any effect in cellular viability *in vitro.* However, after cisplatin treatment, the aptamers A8, A18 and, to a lesser extend, A17 provoked an important increase (3-4 fold increase) in the percentage of cells undergoing apoptosis (see Figures 2A and 2B).

### 4) Aptamers A8 and A18 bind the ATP binding domain of HSP70

In order to determinate with which HSP70 domain are associated the mot efficient apatmers, a mutant of HSP70 that had only the ATP binding domain of the chaperone - i.e. in which HSP70 peptide binding domain has been deleted (RIBEIL et al., Nature, vol.445, p:102-5, 2007)- has been used.

*In vitro* translation of LDH-tagged HSP70 mutant containing only the ATP domain and HA-tagged aptamers was performed using the TNT-coupled reticulocyte lysate system (PROMEGA). For immunoprecipitations, 15 µL of each aptamers reticulocyte lysate was incubated with 15 µL of HSP70 mutant reticulocyte lysate at room temperature for 30 min. Immunoprecipitations were subsequently performed in a final total of 500 µL of immunoprecipitation buffer as described previously except for the use of an anti-LDH antibody (SANTA CRUZ).

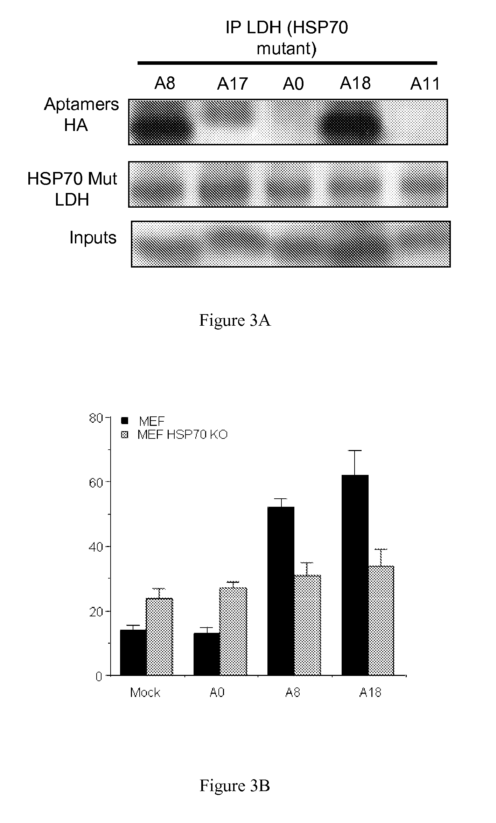

The figure 3A corresponds to the immunoblots showing the signal obtained for HSP70 mut-LDH and for the HA-tag with A8, A17, A0, A18 and A11 aptamers.

The results established that whereas a strong interaction with the ATP binding domain of HSP70 was found for the aptamers A8 and A18, only a weak interaction was detected for A17 (see Figure 3A). Finally, no interaction at all was detected for A11 or the control aptamer A0.

### 5) in vitro chemo-sensitizing properties of A8 and A18 aptamers is HSP70-dependent

Inducible HSP70 belongs to the so-called HSP70 family, which comprises at least 8 homologous proteins. In order to determine the relative contribution of inducible HSP70 to A8- and A18-mediated cell killing, the capacity of these aptamers to kill MEFs (Mouse Embryonic Fibroblast) originating from wild type mice and from mice deficient for the two genes that encode inducible HSP70 (HSP70.1, HSP70.3) has been evaluated.

Wild type MEFs and HSP70.1-/- HSP70.3-/- MEFs were transiently transfected as disclosed previously with A0, A8 and A18 aptamer coding sequence expression vector or mock as a control. Then, the cells were treated with cisplatin as above dislosed, and the percentage of cells with condensate nucleus was determined by staining with Hoechst 33342.

The figure 3B shows the percentage of apoptosis obtained with A0, A8 and A18 aptamer coding sequence expression vector or mock as measured by counting respectively Wild type MEFs (MEF) and HSP70.1-/- HSP70.3-/- MEFs (MEF HSP70 KO) having condensed and fragmented nuclear chromatin after cell staining with Hoechst 33342 dye.

The results show that the expression level of inducible HSP70 had profound effects on the cytotoxic potential of A8 and A18 aptamers. In fact, both peptide aptamers A8 and A18 showed a strong cytotoxic effect on wild type MEFs in combination with cisplatin (see Figure 3B). In sharp contrast, both aptamers proved completely inactive on HSP70.1-/- HSP70.3 -/- MEFs (see Figure 3B). These results establish that the cytotoxic activity of these peptide aptamers is mediated by the specific targeting of inducible HSP70. This is an important observation because it is specifically the inducible HSP70 that is over-expressed in cancer and that is necessary for the illicit survival of tumor cells.

### 6) HSP70-binding peptide aptamers induce tumor regression in vivo.

To determine whether the chemosensitizing effect of A8 and A18 aptamers observed *in vitro* was also detected *in vivo,* mouse B16F10 melanoma cells were stably transfected with the aptamer expression vectors (A8, A18 and, as a control, A0). Stably transfected cells were selected using G418 (SIGMA) according to the manufacturer's instruction.

Exponentially growing B16F10 cells (wild type and aptamers-transfected) were harvested, washed in PBS, and resuspended in RPMI medium without foetal bovine serum (FBS) to a concentration of 2x10⁵ B16F10 cells in 100 µl. B16F10 cells were then injected subcutaneously into the right side of syngeneic C57/B16 mice (9 mice/group). Next, some mice bearing B16F10-A0, B16F10-A8 or B16F10-A18 tumors were treated with cisplatin (10 mg/kg), given intraperitonealy as a single dose at day 6 after tumor cells injection. Tumor volume was evaluated for all mice every two days, using a caliper to measure two perpendicular diameters.

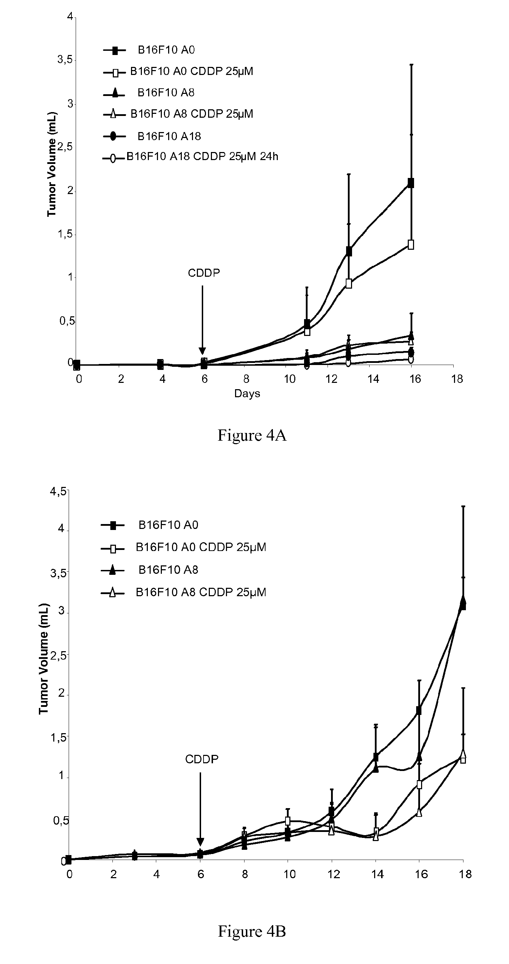

The figure 4A shows the evolution of the tumor volume in C57/B16 mice injected with B16F10-A0 (control), B16F10-A8 or B16F10-A18 untreated (black symbol) or treated with cisplatine (white symbol).

Cells expressing the A0 aptamer, like wild type B16 cells (not shown), formed tumors that rapidly progressed. Surprisingly, the cells expressing the aptamers A8 or A18 gave rise to much smaller tumors that did not progress (see Figure 4A). Thus, A8 and A18 aptamers bear an anti-tumoral activity *in vivo.*

In mice bearing control tumors (wt B16F10 and B16F10-A0) cisplatin treatment slightly reduced the size of the tumors (see Figure 4A). In mice bearing the already small B16-A8 and B16-A18 tumors, cisplatin induced a complete regression of the tumors in most mice (6±1, n=9) (see Figure 4A).

The contribution of the immune system to the anti-tumoral effect of A8 and A18 aptamers was then studied by the duplication of the previous injection experiments into immunodeficient athymic nude (*nu*/*nu*) mice.

The figure 4B shows the evolution of the tumor volume in nude mice injected with B16F10-A0 (control), B16F10-A8 or B16F10-A18 untreated (black symbol) or treated with cisplatine (white symbol).

As shown in Figure 4B, no difference in tumor growth was found among the different injected cells without cisplatin treatment, indicating that the development of an immune response was necessary for the anti-tumor properties of A8 and A18 peptide aptamers. Moreover, the effect of cispatin was the same for the tumors expressing A8, A18 or the aptamer control A0 (Fig. 4B), indicating again that the immune system was involved in the animals' response to A8 and A18 expression.

### 7) A8 and A18 induce an increase in the number of tumor infiltrating CD8⁺ T cells and macrophages.

To further analyze the immune response induced by the peptide aptamers in syngeneic mice, the injected animals were killed 15 days after cell injection. The site of tumor cell injection was resected and snap-frozen in methylbutane that had been cooled in liquid nitrogen. An immunofluorescence stainning study of tumor-infiltrating inflammatory cells was performed on acetone-fixed 5 µm cryostat sections. The slides were washed two times in PBS 1X and one time with the blocking buffer (5% BSA in PBS 1X) and incubated with either normal mouse IgG or rabbit IgG or immune cells specific antibodies diluted in PBS-BSA 1% Saponin 0.1%, one hour at room temperature. The slides were rinsed with PBS 1X and with PBS-BSA 5%, then incubated with the secondary antibodies biotinylated anti-mouse or anti-rabbit, diluted 1/200 in PBS-BSA 5%, 45 minute in the dark. The slides were washed and incubated with a complexe strepavidin alexa 488 (molecular probes) diluted 1/1000 in PBS-BSA 1% Saponin 0.1%, in the dark. Finally, the slides were rinsed with PBS and one time with H₂O, then mounted in medium with Dapi. Two independent experiments were performed in which 4 mice were injected with the different cells (wild type B16F10 and B16F10 transfected with A8, A18 or A0 as a control). Therefore, for each group, the results are in total from 8 mice.

The immunofluorescence results are disclosed in table II.

**Table II: Cellular location of immune cells**

| | B16F10-A0 | | B16F10-A8 | | B16F10-A18 | |
|---|---|---|---|---|---|---|
| | Periphery | Tumor | Periphery | Tumor | Periphery | Tumor |
| CD8+ T-cells | + | +/- | + | +++ | + | +++ |
| Dendritic cells | ++ | + | ++ | +++ | ++ | ++ |
| Macrophages | ++ | + | ++ | ++++ | ++ | +++ |
| CD4+ T-cells | ++ | - | ++ | +/- | ++ | - |
| TCR | +/- | +/- | +/- | + | +/- | + |

| | | | | | | |
|---|---|---|---|---|---|---|
| -, no labeled cells; +/- , 0-10 % labeled cells; +, 10-20 % labeled cells; ++, 20-40 % labeled cells; +++, 40-60 % labeled cells; ++++, 60-80 % labeled cells; +++++, 80-100 % labeled cells. | | | | | | |

When compared with tumor nodules induced by B16F10-wt and B16-A0, those induced by B16-A8 and B16-A18 cells exhibited a stronger infiltration by CD8⁺ T-cells and macrophages (identified as CD3⁺ cells and F4/80⁺ cells respectively) (Table 2). In contrast, there were no significant difference in the number of tumor infiltrating TCR⁺ cells, NK cells, dendritic cells (identified as IA/IE⁺ cells) and CD4⁺ T cells in B16-A8 and B16-A18 tumors as compared to the control tumors (B16F10 and B16-A1) (Table2). These results suggested that CD8⁺ T cells and macrophages that infiltrate B16-A8 and B16-A18 tumors in syngeneic animals may play a role in the decreased tumorigenicity of cells expressing this peptide.

Finally, it has been found that the two most efficient peptide aptamers bind the ATP pocket of HSP70. *In vitro,* this aptamers did not have any toxicity by their own but strongly increased the cells' sensitivity to anticancer agents like cisplatin. *In vivo*, the expression of the aptamers was enough to induce the tumor cells' to die. This anticancer response induced by HSP70 chaperone neutralization involved the immune system of the animal because it was not observed in nude animals. Confirming these results, a massive infiltration of T-cells and macrophages can be detected in the tumors expressing the peptide aptamers, while hardly no infiltration is detected in control tumors.

### 8) Screening for compounds that efficiently bind to HSP70.

In order to identify compounds that efficiently bind to HSP70, and preferably to the ATPase domain of HSP70, the inventors use the aptamers described previously in the two-hybrid interaction assays disclosed in international patent application WO 2005/095645.

Briefly, a TB50α yeast strain is co-transformed with pHB2-ruc comprising a LexA recognition site and ruc as a luciferase, a plasmid directing the expression of LexA-HSP70 or of LexA-HSP70 ATPase domain, and different plasmids directing the expression of HSP70 aptamers fused to an activation domain. Yeasts are then grown from colonies of transformants.

3'-sulfogalactolipids, such as 3'-sulfogalactosyl ceramide (SGC) or 3'-sulfogalactosylglycerolipid (SGG), are known to bind to the ATPase domain of HSP70 (MAMELAK & LINGWOOD, J. Biol. Chem.., vol.276(1), p: 449-456, 2001).

The transformants are incubated with different test compounds, such as aryl sulphates, and with SGG (150 ng) as a control for the binding to the ATPase domain of HSP70.

Then, the expression of peptides aptamers is induced for 4 hours, in 4 different wells per interaction.

The inhibition of two-hybrid signals enables to identify the compounds which specifically bind to HSP70.

## Claims

1. A polypeptide comprising (i) an amino acid sequence from less than 15 amino acids in length, which has the ability to bind to HSP70, wherein said amino acid sequence is selected in the group comprising SEQ ID NO: 9, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19, and derivatives thereof.

2. The polypeptide of claim 1, wherein the amino acid sequence from less than 15 amino acids which has the ability to bind to the ATPase domain of HSP70, and is selected in the group comprising SEQ ID NO: 9, and SEQ ID NO: 19, and derivatives thereof.

3. The polypeptide of any one of claims 1 or 2, wherein said polypeptide further comprises (ii) an amino acid sequence coding for a cell penetrating peptide.

4. A nucleic acid sequence encoding for a polypeptide as defined in any one of claims 1 to 3.

5. A vector comprising a nucleic acid sequence as defined in claim 4.

6. A host cell genetically engineered with the nucleic acid as defined in claim 4 or with the vector as defined in claim 5.

7. A pharmaceutical composition comprising a polypeptide as defined in any one of claims 1 to 3, a nucleic acid encoding thereof, or a nucleic acid vector comprising said nucleic acid, eventually associated with a pharmaceutically acceptable vehicle.

8. The pharmaceutical composition of claim 7, wherein said composition further comprises at least one cytotoxic agent.

9. The pharmaceutical composition of claim 7, wherein said composition does not comprise any cytotoxic agent.

10. Use of a polypeptide as defined in any one of claims 1 to 3, a nucleic acid sequence coding therefore, a vector comprising such a nucleic acid sequence, or a composition as defined in any one of claims 7 to 9 for treating cancer in a subject.

11. A method for screening candidate compounds capable of modulating the intracellular interaction between HSP70 and a polypeptide as defined in any of claims 1 to 3, wherein said method comprises the steps of:
(i) transforming a mammalian cell with an interactive fusion protein construct comprising a constitutive promoter operably linked to an HSP 70 coding sequence fused in frame with a DNA binding domain, a second interactive fusion protein construct comprising an inducible promoter operably linked to a sequence coding for a polypeptide as defined in any of claims 1 to 3 fused in frame with a transcriptional activation domain and a reporter construct comprising a DNA binding element bindable to said DNA binding domain protein operably linked to a minimal promoter and a reporter coding sequence;
(ii) contacting or not a candidate compound with said mammalian cell allowing its contact with the HSP 70 coding sequence,
(iii) inducing the expression of the second interactive fusion protein corresponding to a polypeptide as defined in any of claims 1 to 3 fused in frame with a transcriptional activation domain,
(iv) comparing the expression of the reporter coding sequence in the presence or absence of the contacting step with the candidate compound, and
(v) selecting the candidate compound which induces an inhibition of the expression of the reporter coding sequence.
